# FASCICULE DE BREVET EUROPEEN

(11) **EP 3 013 841 B1**
(45) Date de publication et mention de la délivrance du brevet: **03.05.2017**
(21) Numéro de dépôt: 14749888.5
(22) Date de dépôt: 26.06.2014
(51) Int. Cl.: B01J 31/18, C07C 2/32, C07C 2/36, C07F 15/04, C08F 4/80

(54) **NOUVEAU COMPLEXE À BASE DE NICKEL ET SON UTILISATION DANS UN PROCEDE D'OLIGOMERISATION DES OLEFINES**
NEUARTIGER NICKELBASIERTER KOMPLEX UND VERWENDUNG DAVON IN EINEM VERFAHREN ZUR OLIGOMERISIERUNG VON OLEFINEN
NOVEL NICKEL-BASED COMPLEX AND USE THEREOF IN A METHOD FOR THE OLIGOMERISATION OF OLEFINS

(30) Priorité: 28.06.2013 FR 1356271
(43) Date de publication de la demande: 04.05.2016
(73) Titulaire: IFP Énergies nouvelles, 92852 Rueil-Malmaison (FR); Universiteit van Amsterdam, 1000 GG Amsterdam (NL)
(72) Inventeur: BREUIL, Pierre-Alain, Lyon 69006 (FR); BOULENS, Pierre, F-PARIS 75017 (FR); REEK, Joost, NL-3817 BK Amersfoort (NL); OLIVIER-BOURBIGOU, Helene, F-69230 Saint Genis-laval (FR)
(86) Numéro de dépôt international: PCT/FR2014/051625
(87) Numéro de publication internationale: WO 2014/207393

(56) Documents cités:
- ANAGHO L E ET AL: "Synthesis and Solid-State Structure of a Metal Complex of a Diphosphineimine", ANGEWANDTE CHEMIE INTERNATIONAL EDITION, WILEY - V C H VERLAG GMBH & CO. KGAA, DE, vol. 44, 1 janvier 2005 (2005-01-01), pages 3271-3275, XP002434506, ISSN: 1433-7851, DOI: 10.1002/ANIE.200462588
- KEMING SONG ET AL: "Syntheses, Structures, and Catalytic Ethylene Oligomerization Behaviors of Bis(phosphanyl)aminenickel(II) Complexes Containing N-Functionalized Pendant Groups", EUROPEAN JOURNAL OF INORGANIC CHEMISTRY, vol. 2009, no. 20, 5 juillet 2009 (2009-07-05), pages 3016-3024, XP055101476, ISSN: 1434-1948, DOI: 10.1002/ejic.200900256 cité dans la demande

## Description

La présente invention concerne une nouvelle famille de complexes de nickel, et leurs méthodes de préparation. L'invention concerne également l'utilisation desdits complexes comme catalyseurs de réactions de transformations chimiques.

### Art antérieur :

Il est connu de préparer des complexes à base de nickel pour leur application dans divers domaines de la chimie, notamment dans le domaine des transformations catalytiques tels que l'hydroformylation, l'hydrogénation, le couplage croisé, l'oligomérisation des oléfines...

Parmi ces complexes, on peut citer à titre d'exemple l'article C.R. Acad. Sci. 1967, C103-106 et l'article J. Mol. Catal. A 2001, 169, 19-25 dans lesquels sont décrits des complexes de nickel en présence de monophosphine.

L. E. Anagho et al. décrivent dans Angew. Chem. Int. Ed. Engl. 2005, 44, 3271-3275 des complexes de Nickel à base d'un ligand diphosphoshineimine. Les complexes de nickel-diphosphinamines décrits dans l'art antérieur sont symétriques et préparés au moyen de ligands diphosphinamines dans lesquels les deux atomes de phosphore sont porteurs de groupements identiques de nature aromatique (Eur. J. Inorg. Chem, 2009, 3016-3024, Organometallics, 2001, 20, 4769-4771). Par exemple, la demande de brevet WO01/10876 décrit des complexes de nickel diphosphinamines, les ligands symétriques décrits étant substitués, sur les phosphores, uniquement par des groupements aromatiques et utilisés pour la polymérisation de l'éthylène.

Ces systèmes catalytiques sont peu actifs en oligomérisation de l'éthylène et sont généralement utilisés pour la polymérisation de l'éthylène.

La demanderesse a trouvé un nouveau complexe dissymétrique de nickel préparé à partir de ligands diphosphinamines ou iminobisphosphines dissymétriques, dans lesquels un des atomes de phosphore porte au moins un groupement non aromatique et l'autre atome de phosphore porte au moins un groupement aromatique. Il a été trouvé que lesdits complexes, en présence d'un solvant ou pas, présentent une activité et une sélectivité améliorées pour les réactions de transformation catalytique, en particulier pour la catalyse de la réaction d'oligomérisation ou de dimérisation des oléfines.

### Description détaillée de l'invention

### Complexe de nickel de formule (I)

Un premier objet de l'invention concerne un nouveau complexe dissymétrique de nickel de formule (I) : 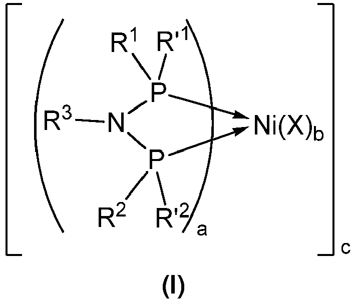 dans lequel
- les groupements R¹ et R'¹, identiques ou différents, liés ou non entre eux, sont choisis parmi les groupements non aromatiques et ne contenant pas de silicium.
- les groupements R² et R'², identiques ou différents, liés ou non entre eux, sont choisis parmi les groupements aromatiques,
- R³ est choisi parmi l'hydrogène, les halogènes, les groupements hydrocarbonés aliphatiques, cycliques ou non, contenant ou non des hétéroéléments, les groupements aromatiques contenant ou non des hétéroéléments, substitués ou non,
- X est un anion ou un donneur d'électrons, les groupements X peuvent être liés entre eux ou non, X est choisi parmi l'hydrogène, les halogènes, les groupements hydrocarbonés aliphatiques, cycliques ou non, contenant ou non des hétéroéléments substitués ou non, les groupements aromatiques contenant ou non des hétéroéléments, substitués ou non, les oléfines contenant ou non des hétéroéléments, substituées ou non, les borates, les phosphates, les sulfates, les ligands phosphorés contenant ou non des hétéroéléments, substitués ou non, les groupements -OR⁴ ou -N(R⁵)(R⁶), où R⁴, R⁵ et R⁶ sont choisis parmi les groupements hydrocarbonés aliphatiques, cycliques, contenant ou non des hétéroéléments, les groupements aromatiques contenant ou non des hétéroéléments, substitués ou non, - a est un nombre entier compris entre 1 et 4, b est un nombre entier compris entre 0 et 6, c est un nombre entier compris entre 1 et 4.

Les groupements R¹ et R'¹ sont choisis parmi les groupements non aromatiques et ne contenant pas de silicium. De préférence, R¹ et R'¹ sont identiques.

De préférence, les groupements R¹ et R'¹ sont choisis parmi les groupements méthyle, éthyle, isopropyle, *n*-butyle, *iso*-butyle, *tert*-butyle, pentyle, cyclohexyle, substitués ou non.

De préférence, les groupements R² et R'² sont choisis parmi les groupements phényle, o-tolyl, m-tolyl, *p*-tolyl, mésityle, 3,5-diméthylphényle, 4-méthoxyphényle, 2-méthoxyphényle, 2-isopropoxyphényle, 4-méthoxy-3,5-diméthylphényle, 3,5-ditert-butyl-4-méthoxyphényle, 3,5-bis(trifluorométhyl)phenyle, benzyle, naphthyle, pyridyle, substitués ou non, contenant des hétéro-éléments ou non. De préférence, R² et R'² sont identiques.

Avantageusement, R³ est choisi parmi l'hydrogène, les groupements alcoxy, aryloxy, sulfure, sulfonamino, sulfonamido, nitro, carbonyle, amino, amido comprenant ou non des groupements aliphatiques, cycliques ou aromatiques, contenant ou non des hétéroéléments, substitués ou non.

Le complexe de formule (I) est avantageusement préparé en mettant en contact un précurseur de nickel A et au moins un ligand diphosphinamine B1 de formule (R¹)(R'¹)P-N(R³)-P(R²)(R'²) ou un ligand iminobisphosphine B2 de formule (R³)N=P(R¹)(R'¹)-P(R²)(R'²) en présence ou pas d'un solvant, appelé solvant de préparation, à une température comprise entre -80°C et +110°C, pendant une durée comprise entre 1 minute et 24 heures.

Le précurseur de nickel A peut être choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), le nickel bis(éthylène), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange. Lesdits précurseurs de nickel peuvent éventuellement être complexés à des bases de Lewis.

Le solvant de préparation peut être choisi parmi les solvants organiques et de préférence parmi les éthers, les alcools, les solvants chlorés et les hydrocarbures saturés, insaturés, aromatiques ou non, cycliques ou non. De préférence, ledit solvant préparation est choisi parmi l'hexane, le cyclohexane, le méthylecyclohexane, l'heptane, le butane ou l'isobutane, les monooléfines ou dioléfines comportant de préférence 4 à 20 atomes de carbone, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où ledit solvant est un liquide ionique, il est avantageusement choisi parmi les liquides ioniques décrits dans les brevets US 6,951,831 B2 et FR 2895406 B1.

La préparation des ligands diphosphinamines B1 de formule (R¹)(R'¹)P-N(R³)-P(R²)(R'²), ou iminobisphosphines B2 de formule (R³)N=P(R¹)(R'¹)-P(R²)(R'²) s'effectue selon les méthodes connues de la littérature (Inorg. Chem. 2003, 2125-2130). Les ligands diphosphinamines B1 de formule (R¹)(R'¹)P-N(R³)-P(R²)(R'²) peuvent être préparés et isolés en faisant réagir 1 équivalent de chlorophosphine Cl-P(R¹)(R'¹) et 1 équivalent de chlorophosphine Cl-P(R²)(R'²) avec une amine primaire ou aromatique R³-NH₂ en présence de triéthylamine. Les ligands iminobisphosphines B2 de formule (R³)N=P(R¹)(R'¹)-P(R²)(R'²) peuvent être préparés et isolés en faisant réagir une amine primaire ou aromatique R³-NH₂ et 1 équivalent de chlorophosphine Cl-P(R¹)(R'¹) et 1 équivalent de chlorophosphine Cl-P(R²)(R'²) introduits successivement en présence de triethylamine.

### Utilisation du complexe de formule (I) dans une réaction de transformation chimique

Le complexe de nickel de formule (I) selon l'invention peut être utilisé comme catalyseur dans une réaction de transformation chimique, telle que la réaction d'hydrogénation, d'hydroformylation, de couplage croisé ou d'oligomérisation des oléfines. En particulier, le complexe de nickel de formule (I) est utilisé dans un procédé d'oligomérisation des oléfines comportant avantageusement 2 à 10 atomes de carbone; de préférence dans un procédé de dimérisation de l'éthylène ou du propylène.

Le complexe de nickel de formule (I) selon l'invention peut être utilisé sous forme de composition catalytique, en mélange avec un composé C appelé agent activateur. Ledit agent activateur est avantageusement choisi dans le groupe formé par les composés tris(hydrocarbyl)aluminium, les composés chlorés ou bromés d'hydrocarbylaluminium, les halogénures d'aluminium, les aluminoxanes, les composés organo-borés, les composés organiques susceptibles de donner ou de capter un proton, pris seuls ou en mélange.

Les tris(hydrocarbyl)aluminium, les composés chlorés ou bromés d'hydrocarbylaluminium et les halogénures d'aluminium répondent de préférence à la formule générale AlₓR_{y}W_{z} dans laquelle R représente un radical hydrocarboné monovalent contenant par exemple jusqu'à 12 atomes de carbone tel que alkyle, aryle, aralkyle, alkaryle ou cycloalkyle, W représente un atome d'halogène choisi par exemple parmi le chlore et le brome, W étant de préférence un atome de chlore, x prend une valeur de 1 à 2, y et z prennent une valeur de 0 à 3. Comme exemples de tels composés, on peut mentionner le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), le triméthylaluminium, le tributylaluminium, le tri-*n*-octylaluminium et le triéthylaluminium (AlEt₃).

Dans le cas où ledit agent activateur est choisi parmi les aluminoxanes, ledit agent activateur est avantageusement choisi parmi le méthylaluminoxane (MAO), l'éthylaluminoxane et les méthylaluminoxanes modifiés (MMAO). Ces agents activateurs peuvent être utilisés seuls ou en mélange.

De préférence, ledit agent activateur C est choisi parmi le dichloroéthylaluminium (EtAlCl₂) et le méthylaluminoxane (MAO).

Dans le cas où ledit agent activateur est choisi parmi les composés organoborés, ledit agent activateur est de préférence choisi parmi les acides de Lewis de type tris(aryl)borane tels que le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés et les (aryl)borates associés à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.

Dans le cas où ledit agent activateur est choisi parmi les composés organiques susceptibles de donner un proton, ledit agent activateur est de préférence choisi parmi les acides de formule HY dans lequel Y représente un anion.

Dans le cas où ledit agent activateur est choisi parmi les composés organiques susceptibles de capter un proton, ledit agent activateur est de préférence choisi parmi les bases de Bronsted.

Le complexe de nickel de formule (I) selon l'invention ou la composition catalytique le comprenant est avantageusement utilisé dans un procédé d'oligomérisation ou de dimérisation des oléfines, de préférence dans un procédé de dimérisation de l'éthylène ou du propylène.

Le solvant du procédé d'oligomérisation ou de dimérisation peut être choisi parmi les solvants organiques et de préférence parmi les éthers, les alcools, les solvants chlorés et les hydrocarbures saturés, insaturés, aromatiques ou non, cycliques ou non. En particulier, ledit solvant est choisi parmi l'hexane, le cyclohexane, le méthylecyclohexane, l'heptane, le butane ou l'isobutane, les monooléfines ou dioléfines comportant de préférence 4 à 20 atomes de carbone, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où ledit solvant de réaction est un liquide ionique, il est avantageusement choisi parmi les liquides ioniques décrits dans les brevets US 6,951,831 B2 et FR 2895406 B1.

L'oligomérisation est définie comme la transformation d'une unité monomère en un composé ou mélange de composés de formule générale CpH2p avec 4 ≤ p ≤ 80, de préférence avec 4 ≤ p ≤ 50, de manière préférée avec 4 ≤ p ≤ 26 et de manière plus préférée avec 4 ≤ p ≤ 14.

Les oléfines utilisées dans le procédé d'oligomérisation ou de dimérisation sont des oléfines comportant de 2 à 10 atomes de carbone. De préférence, lesdites oléfines sont choisies parmi l'éthylène, le propylène, les n-butènes et les n-pentènes, seules ou en mélange, pures ou diluées.

Dans le cas où lesdites oléfines sont diluées, lesdites oléfines sont diluées par un ou plusieurs alcane(s), tels qu'on les trouve dans des « coupes » issues des procédés de raffinage du pétrole, comme le craquage catalytique ou le craquage à la vapeur.

De manière préférée, l'oléfine utilisée dans le procédé d'oligomérisation ou de dimérisation est de l'éthylène ou du propylène.

Lesdites oléfines peuvent venir de ressources non fossiles telles que la biomasse. Par exemple, les oléfines utilisées dans le procédé d'oligomérisation selon l'invention peuvent être produites à partir d'alcools, et en particulier par déshydratation des alcools.

La concentration du nickel dans la solution catalytique est avantageusement comprise entre 1.10⁻⁸ et 1 mol/L, et de préférence entre 1.10⁻⁶ et 1.10⁻² mol/L.

Le rapport molaire entre l'agent activateur C et le complexe de nickel est avantageusement compris entre 1/1 et 10000/1, de préférence entre 1/1 et 1000/1 pour les aluminoxanes et de préférence entre 1/1 et 100/1 pour les autres dérivés d'aluminium et les autres acides de Lewis.

Le procédé d'oligomérisation ou de dimérisation opère avantageusement à une pression totale comprise entre la pression atmosphérique et 20 MPa, de préférence entre 0,5 et 8 MPa, et à une température comprise entre -40 et +250°C, de préférence entre -20°C et 150°C.

La chaleur engendrée par la réaction peut être éliminée par tous les moyens connus de l'homme du métier.

Le procédé d'oligomérisation ou de dimérisation peut être conduit en système fermé, en système semi-ouvert ou en continu, avec un ou plusieurs étages de réaction. Une vigoureuse agitation est avantageusement mise en oeuvre pour assurer un bon contact entre le ou les réactifs et la composition catalytique.

Le procédé d'oligomérisation ou de dimérisation peut être mis en oeuvre en discontinu. Dans ce cas, un volume choisi de la solution comprenant le complexe selon l'invention est introduit dans un réacteur muni des dispositifs habituels d'agitation, de chauffage et de refroidissement.

Le procédé d'oligomérisation ou de dimérisation peut également être mis en oeuvre en continu. Dans ce cas, la solution comprenant le complexe selon l'invention est injectée en même temps que l'oléfine dans un réacteur agité par les moyens mécaniques classiques ou par une recirculation extérieure, et maintenue à la température souhaitée.

La composition catalytique est détruite par tout moyen habituel connu par l'homme du métier, puis les produits de la réaction ainsi que le solvant sont séparés, par exemple par distillation. L'oléfine qui n'a pas été transformée peut être recyclée dans le réacteur.

Le procédé selon l'invention peut être mis en oeuvre dans un réacteur à un ou plusieurs étages de réaction en série, la charge oléfinique et/ou la composition catalytique au préalable pré-conditionnée étant introduites en continu, soit dans le premier étage, soit dans le premier et un autre quelconque des étages. A la sortie du réacteur, la composition catalytique peut être désactivée, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Les produits du présent procédé peuvent trouver une application par exemple comme composants de carburants pour automobiles, comme charges dans un procédé d'hydroformylation pour la synthèse d'aldéhydes et d'alcools, comme composants pour l'industrie chimique, pharmaceutique ou la parfumerie et/ou comme charges dans un procédé de métathèse pour la synthèse de propylène par exemple.

Les exemples suivants illustrent l'invention sans en limiter la portée. La notation Cy représente le groupement tricyclohéxyle.

### Exemple 1:

### Synthèse des ligands

Des ligands iminobisphosphines R'-SO₂-N=P(R¹)(R'¹)-P(R²)(R'²) sont préparés et isolés en faisant réagir un sulfonamide et 2 équivalents de chlorophosphine (identiques ou différents) en présence de triéthylamine. On exemplifie les ligands **1** et **2** dans lesquels R¹=R'¹=R²=R'² (exemples comparatifs) et les ligands **3** et **4** dans lesquels R¹=R'¹ et R²=R'² et R¹ est différent de R². Les structures des quatre ligands sont représentées ci-dessous.

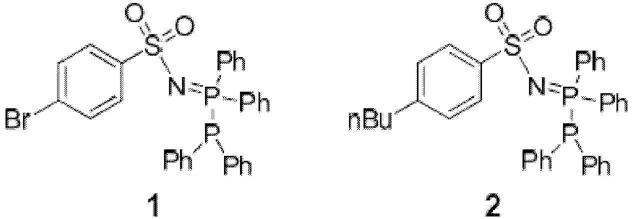

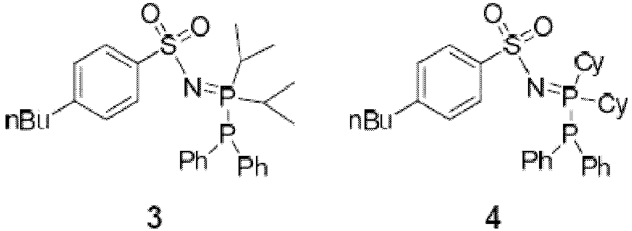

### Synthèse du ligand 1 : (4-bromo-N-(1,1,2,2-tetraphenyldiphosphanylidene) benzenesulfonamide)

A une solution de 4-bromobenzenesulfonamide (500 mg, 2.12 mmol, 1 eq.) et de triéthylamine (1.6 mL, 11.2 mmol, 5.3 eq.) dans le THF (10mL) est ajoutée goutte-à-goutte, à température ambiante et sous forte agitation la chlorodiphénylphosphine (0.760 mL, 4.24 mmol, 2 eq.) fraîchement distillée. Une fois l'addition terminée, le mélange est agité pendant 5 minutes puis la suspension est filtrée sous atmosphère d'azote sur un fritté. L'évaporation du solvant et des volatiles conduit à la formation d'un solide. Ce solide est dissous dans un minimum de dichlorométhane, puis du pentane (20 mL) est ajouté. En évaporant cette solution, un précipité apparaît. Le surnageant est retiré à l'aide d'une seringue puis le solide est lavé au pentane (2x10 mL) et séché sous vide pour donner le ligand **1** sous la forme d'une poudre blanche (rendement isolé : 68%).

¹H NMR (300 MHz, CD₂Cl₂): δ 7.82 - 6.89 (m, 24H). ³¹P NMR (121 MHz, CD₂Cl₂) 19.72 (d, *J* = 281.1 Hz), -18.74 (d, *J* = 281.1 Hz).

³¹P{¹H} NMR (121 MHz, CD₂Cl₂): 19.72 (d, *J* = 279.9 Hz), -18.74 (d, *J* = 281.2 Hz).

MS(FAB⁺): m/z calc. pour C₃₀H₂₅NO₂P₂BrS ([MH]⁺) : 606,0248; obs.: 606,0255.

### Synthèse du ligand 2 : (4-butyl-N-(1,1,2,2-tetraphenyldiphosphanylidene) benzenesulfonamide)

A une solution de 4-butylbenzenesulfonamide (500 mg, 2.34 mmol, 1 eq.) et de triéthylamine (1 mL, 7.17 mmol, 3 eq.) dans le THF (20 mL) est ajoutée goutte-à-goutte, à température ambiante et sous forte agitation, la chlorodiphénylphosphine fraichement distillée (0.840 mL, 4.68 mmol, 2 eq.). Une fois l'addition terminée, le mélange est agité pendant 5 minutes puis la suspension est filtrée sous atmosphère d'azote sur un fritté. L'évaporation du solvant et des volatiles conduit à la formation d'une huile. Cette huile est solubilisée dans de l'éther diéthylique (10 mL) et la solution est évaporée. Cette étape est répétée 4 fois jusqu'à ce que le produit précipite. Le solide est alors séché sous vide pour obtenir le ligand **2** sous la forme d'une poudre blanche (rendement isolé: 79%).

¹H NMR (300 MHz, CD₂Cl₂): δ 7.91 - 6.33 (m, -CH_{Ar}, 24H), 2.59 (t, CH₃-CH₂-CH₂-CH₂-CAr, *J* = 7.7 Hz, 2H), 1.57 (m, CH₃-CH₂-CH₂-CH₂-CAr, 2H), 1.33 (m, CH₃-CH₂-CH₂-CH₂-Car, 2H), 0.93 (t, CH₃-CH₂-CH₂-CH₂-Car, *J* = 7.3 Hz, 3H).

³¹P NMR (121 MHz, CD₂Cl₂): δ 19.47 (d, *J* = 277.9 Hz), -17.90 (d, *J* = 278.0 Hz).

MS (FAB+) : m/z calc. pour C₃₄H₃₄O₂NP₂S ([M+H]⁺): 582.1786 ; obs.: 582.1790

### Synthèse du N-diphénylphosphino-4-butylbenzenesulfonamide

A une solution de 4-butylbenzene-1-sulfonamide (9.38 mmol, 1 eq.) et de triéthylamine (25 mmol) dans le THF (20 mL) est ajoutée goutte-à-goutte, à température ambiante et sous forte agitation, la chlorodiphénylphosphine fraichement distillée (9.38 mmol, 1 eq.). La suspension est laissée sous agitation, une nuit à température ambiante. L'évaporation du solvant et des volatiles conduit à la formation d'un solide. Ce solide est dissous dans 10 mL de dichlorométhane, puis du pentane (40 mL) est ajouté, un précipité apparaît. Le surnageant est retiré à l'aide d'une seringue puis le solide est lavé au pentane (2x20 mL) et séché sous vide pour donner le N-diphénylphosphino-4-butylbenzenesulfonamide sous la forme d'une poudre blanche. Ce composé peut être isolé et purifié ou utilisé directement pour une autre étape de synthèse (rendement isolé : 74%).

### Synthèse du ligand 3 : 4-butyl-N-(1,1-diisopropyl-2,2-diphenyldiphosphanylidene) benzenesulfonamide

A une solution de N-diphénylphosphino-4-butylbenzenesulfonamide (1.86 g, 4.68 mmol, 1 eq.) et de triéthylamine (1.30 mL, 9.36 mmol, 2 eq.) dans le THF (20 mL), est ajoutée goutte-à-goutte, à température ambiante et sous forte agitation, la diisopropylchlorophosphine (0.746 mL, 4.68 mmol, 1 eq.). Une fois l'addition terminée, le mélange est agité pendant 10 minutes puis la suspension est filtrée sous atmosphère d'azote sur un fritté. L'évaporation du solvant et des volatiles conduit à la formation d'une huile. Du pentane (20 mL) est ajouté à cette huile, puis après trituration le pentane est retiré à l'aide d'une seringue. L'huile est alors mise en suspension dans le pentane (10 mL) et la solution évaporée sous vide. Cette étape est répétée une fois avec du pentane puis deux fois avec de l'éther diéthylique (10 mL) ce qui permet la formation d'un solide. Le solide est lavé avec du pentane (2 x 10 mL) puis séché sous vide pour donner le ligand **3** sous forme d'un solide blanc (rendement isolé : 34%).

¹H (300 MHz, CD₂Cl₂): δ: δ 7.98 - 7.83 (m, 4H, -PPh₂), 7.76 - 7.64 (m, 2H,-CH₂-Ar-SO₂), 7.60 - 7.35 (m, 6H, -PPh₂), 7.23 - 7.12 (m, 2H, -CH₂-Ar-SO₂), 2.69 - 2.57 (t, 2H, J=7.4 Hz, CH₃-CH₂-CH₂-CH₂-Ar), 2.44 (m, 2H, CH₃-CH-CH₃), 1.69 - 1.48 (m, 2H, CH₃-CH₂-CH₂-CH₂-Ar), 1.35 (m, 2H, CH₃-CH₂-CH₂-CH₂-Ar), 1.18 - 0.99 (m, 12H, CH₃-CH-CH₃), 0.93 (t, J = 7.3 Hz, 3H, CH₃-CH₂-CH₂-CH₂-Ar).

³¹P(121 MHz, CD₂Cl₂) δ: 20.13 (d, J=311.6 Hz) ; 2.80 (d, J= 311.6 Hz).

### Synthèse du ligand 4 : 4-butyl-N-(1,1-dicyclohexyl-2,2-diphenyldiphosphanylidene)benzenesulfonamide

A une solution de -diphénylphosphino-4-butylbenzenesulfonamide (0.361 g, 0.91 mmol, 1 eq.) et de triéthylamine (0.126 mL, 1.82 mmol, 2 eq.) dans le THF (10 mL), est ajoutée goutte-à-goutte, à température ambiante et sous forte agitation, la dicyclohexylylphosphine (0.200 mL, 0.91 mmol, 1 eq.). Une fois l'addition terminée, le mélange est agité pendant 5 minutes puis la suspension est filtrée sous atmosphère d'azote sur un fritté. L'évaporation du solvant et des volatiles conduit à la formation d'une huile. Du pentane (10 mL) est ajouté à cette huile, puis après trituration, il est évaporé sous vide. Cette étape est répétée une fois avec du pentane puis deux fois avec de l'éther diéthylique (10 mL) ce qui permet la formation d'un solide. Le solide est séché sous vide pour donner le ligand **4** sous forme d'un solide blanc (rendement isolé : 51 %).

¹H NMR (300 MHz, CD₂Cl₂): δ 7.90 (dd, *J* = 12.5, 7.6 Hz, 4H, PPh₂), 7.78 - 7.67 (dd, *J* = 8.4, 2.0 Hz, 2H, Ar-SO₂), 7.61 - 7.40 (m, 6H, PPh₂), 7.18 (dd, *J* = 8.4, 2.0 Hz, 2H, Ar-SO₂), 2.63 (t, *J* = 7.6 Hz, 2H, -CH₂-Ar), 2.30 - 2.01 (m, 2H, Cy), 1.81 (m, 2H, Cy), 1.73 - 1.49 (m, 8H, Cy), 1.73 - 1.49 (m, 2H, -CH₂-CH₂-Ar) 1.33 (dt, *J* = 16.3, 7.3 Hz, 2H, -CH₂-CH₂-CH₂-Ar), 1.17 (m, 10H, Cy), 0.93 (t, *J* = 7.3 Hz, 3H, H₃C-CH₂-CH₂).

³¹P NMR (121 MHz, CD₂Cl₂): δ 20.44 (d, *J* = 314.9 Hz), -4.98 (d, *J* = 314.4 Hz).

MS (FAB+) : m/z calcd. For C₃₄H₃₄O₂NP₂S ([M+H]⁺): 594.2725 ; obsd.: 594.2732.

### Synthèse des complexes de nickel

Les ligands **1, 2, 3** et **4** sont mis à réagir avec du NiBr₂(dme) pour donner les complexes **5, 6, 7** et **8.** Le complexe **9** est un complexe de référence.

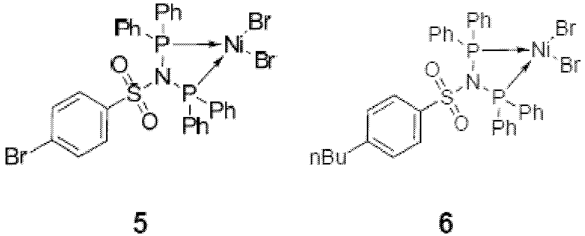

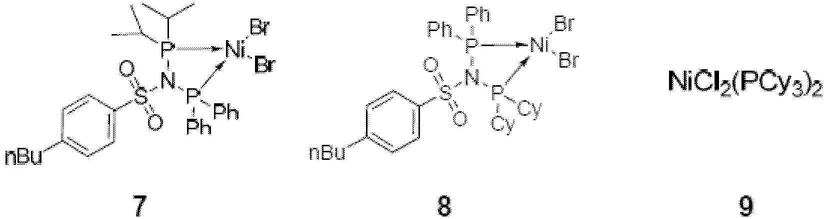

### Synthèse du complexe 5 (comparatif)

Du 4-bromo-N-(1,1,2,2-tetraphenyldiphosphanylidene)benzenesulfonamide **1** (200 mg, 0.331 mmol, 1.01 eq) et du bromure de nickel(II) diméthoxyethane (101 mg, 0.327 mmol, 1 eq) sont mis en suspension dans le toluène (3 mL). La suspension est agitée à 60°C pendant 2 heures. Après refroidissement, le solvant est retiré à l'aide d'une seringue et le solide lavé trois fois avec du pentane (5 mL) puis séché sous vide pour donner le complexe **5** sous forme d'une poudre rouge-marron (rendement isolé: 80 %). Le produit est analysé par masse, RMN du proton et du phosphore.

¹H NMR (300 MHz, CD₂Cl₂): δ 8.16 (m, 8H, PPh₂), 7.76 (t, *J* = 7.3 Hz, 4Hₚₐᵣₐ), 7.59 (m, 8H, PPh₂), 7.06 (d, *J* = 8.2 Hz, 2H, -Ar-SO₂), 6.18 (d, *J* = 8.5 Hz, 2H, -Ar-SO2).

³¹P NMR (121 MHz, CD₂Cl₂): δ 65.52 . ³¹P{¹H} NMR (121 MHz, CD₂Cl₂) δ 65.52.

MS(FAB⁺): m/z calc. for C₃₀H₂₄NO₂P₂Br₂SNi ([M- HBr]⁺) : 741.8701; obs.: 741.8702.

### Synthèse du complexe 6 (comparatif)

Du 4-butyl-N-(1,1,2,2-tetraphenyldiphosphanylidene)benzenesulfonamide **2** (200 mg, 0.344 mmol, 1 eq) et du bromure de nickel(II) diméthoxyethane (106 mg, 0.344 mmol, 1 eq) sont mis en suspension dans le toluène (3 mL). La suspension est agitée à 65°C pendant 1 h jusqu'à la formation d'un solide rouge. Après refroidissement, le solvant est retiré à l'aide d'une seringue et le solide lavé trois fois avec du pentane (5 mL) puis séché sous vide pour donner le complexe **6** sous forme d'une poudre rouge-marron (rendement isolé: 68 %). Le produit est analysé par RMN du proton et du phosphore.

¹H NMR (300 MHz, CD₂Cl₂): δ 8.91 - 5.91 (m, H_{Ar}, 24H), 2.72 - 2.24 (m, CH₃-CH₂-CH₂-CH₂-CAr 2H), 1.51 (m, CH₃-CH₂-CH₂-CH₂-CAr,2H), 1.31 (m, CH₃-CH₂-CH₂-CH₂-CAr 2H), 0.95 (m, CH₃-CH₂-CH₂-CH₂-CAr 3H).

³¹P NMR (121 MHz, CD₂Cl₂) δ 64.10 . ³¹P{¹H} NMR (121 MHz, CD₂Cl₂) δ 64.07.

### Synthèse du complexe 7

Du 4-butyl-N-(1,1-diisopropyl-2,2-diphenyldiphosphanylidene)benzenesulfonamide **3** (400 mg, 0.786 mmol, 1 eq) et du bromure de nickel (II) diméthoxyethane (266 mg, 0.864 mmol, 1.1 eq) sont mis en suspension dans du dichlorométhane (5 mL) à température ambiante pendant deux heures. La solution est filtrée sur un fritté et le filtrat est évaporé sous vide. Le solide est lavé trois fois avec du pentane (10 mL) et séché sous vide pour donner le complexe **7** sous forme d'une poudre rouge (rendement isolé: 61 %).

¹H NMR (300 MHz, CD₂Cl₂): δ 8.77 - 6.21 (m, 14H, Ar), 3.29 (m, 4H, CH₃-CH-CH₃ and -CH₂-Ar), 2.43 (m, 2H, CH₂-CH₂-Ar), 1.66 - 1.00 (m, 14H, CH₃-CH-CH₃ and CH₃-CH₂-CH₂-CH₂-Ar), 0.78 (t, *J* = 7.8 Hz, 3H, CH₃-CH₂-CH₂-CH₂-Ar).

³¹P NMR (121 MHz, CDCl₃) δ 116.96 (d, *J* = 119.0 Hz), 62.84 (d, *J* = 119.4 Hz).

³¹P{¹H} NMR (121 MHz, CDCl₃) δ 116.92 (d, *J* = 116.5 Hz), 62.84 (d, *J* = 117.6 Hz).

### Synthèse du complexe 8

Du 4-butyl-N-(1,1-dicyclohexyl-2,2-diphenyldiphosphanylidene)benzenesulfonamide **4** (98 mg, 0.165 mmol, 1.02 eq) et du bromure de nickel (II) diméthoxyethane (50 mg, 0.162 mmol, 1 eq) sont mis en suspension dans le dichlorométhane à température ambiante pendant deux heures. La solution est filtrée sur fritté et le filtrat est évaporé sous vide. Le solide est lavé trois fois avec du pentane (5 mL) et séché sous vide pour donner le complexe **8** sous forme d'une poudre rouge (rendement isolé: 54 %). Des cristaux du composé **8** sont obtenus par diffusion lente de pentane dans une solution de **8** dans le toluène et le dichlorométhane. Le produit est analysé par masse, RMN du phosphore et diffraction aux rayons X.

³¹P NMR (121 MHz, CD2Cl2) δ 108.15 (d, *J* = 118.6 Hz), 60.28 (d, *J* = 119.2 Hz). MS (FAB⁺): m/z calc. for C₃₄H₄₅Br₂NO₂P₂SNi ([M]⁺) : 811.0354; obs.: 811.0337.

### Exemple 2. Oligomérisation de l'éthylène

La réaction d'oligomérisation de l'éthylène a été évaluée avec les complexes de nickel **5** et **6** et **7** en présence de méthylaluminoxane (MAO) à 45°C et sous 30 bar d'éthylène (1bar = 0,1 MPa)

Conditions opératoires : Le réacteur de 100 mL est séché sous vide à 100°C pendant 2 heures et mis sous pression d'éthylène. Le catalyseur est introduit (0,1 mmol dans 8 mL de toluène) suivi par le méthylaluminoxane (2 mL, 10% dans le toluène, 300 éq.). La température et la pression sont fixées à 45°C et 35 bar. L'agitation est mise en route (t=0). Après le temps de réaction défini, le réacteur est refroidi à température ambiante et dépressurisé sous agitation. La phase liquide est neutralisée avec H₂SO₄ aqueux et est analysée par GC.

Les complexes **5** et **6** activés par le MAO (300 éq.) sont considérés comme inactifs, la consommation d'éthylène est négligeable. Le complexe **7,** activé par le MAO est très actif en oligomérisation de l'éthylène (> 10⁷ g_{C2H4}/(mol_{Ni}.h)) et aucun polymère n'est formé. Les analyses GC confirment que les produits formés sont principalement des butènes et des hexènes. Les résultats figurent dans le tableau 1.

**Tableau 1.Oligomérisation de l'éthylène catalysée par 5, 6, 7.^{a}**

| Entrée | Complexe | Temps (min.) | Conso. C₂H₄ (g) | Activité^{b} | Distribution en oligomères [% pds] | | | | |
|---|---|---|---|---|---|---|---|---|---|
| | | | | | C4^{c} | C6^{c} | C8^{+c} | 1-C4^{d} | 2-C4^{d} |
| 1^{e} | **5** | 22 | n.d.^{f} | - | - | - | - | - | - |
| 2^{e} | **6** | 20 | n.d.^{f} | - | - | - | - | - | - |
| 3 | **7** | 14 | 31,5 | 14.10⁶ | 60,3 | 25,7 | 14,0 | 6,6 | 93,4 |

| | | | | | | | | | |
|---|---|---|---|---|---|---|---|---|---|
| ^{a} Conditions de réaction : nₙᵢ =10 µmol, co-catalyseur : MAO (300 éq.), 30 bar C₂H₄, 45°C, solvant : toluène (10 mL). ^{b} g_{C2H4}/(mol_{Ni}.h). ^{c} Déterminé par GC, % pds / tous les oligomères. ^{d} % pds / aux autres produits de la coupe C4. ^{e} Exemples comparatifs. ^{f} non déterminé: consommation d'éthylène négligeable, activités observées < 0,7. 10⁶. | | | | | | | | | |

Le complexe **7** dissymétrique conduit à des performances très supérieures en termes d'activité par rapport aux complexes symétriques **5** ou **6.**

### Exemple 3. Oligomérisation du propylène

L'oligomérisation du propylène a été réalisée avec deux activateurs différents : l'EADC (dichlorure d'éthylaluminium) et le MAO (méthylaluminoxane). Les tests réalisés avec le catalyseur **9** NiCl₂(PCy₃)₂ sont des tests de référence.

### Tests avec EADC

Conditions opératoires : Le réacteur de 250 mL est séché sous vide à 100°C pendant 2 heures, refroidi à 10°C puis rempli de propylène (pression de 1,4 bar). 33 mL de chlorobenzène et 10 mL of n-heptane (étalon interne pesé avec précision) sont alors introduits, suivis de 8 g de propylène. Sous agitation, le réacteur est refroidit à -10°C. L'activateur EADC (dichlorure d'éthylaluminium, 0,075 M dans le toluène, 15 éq, 2 mL) est alors injecté, suivi du catalyseur (0.1 mmol dans 5 mL de chlorobenzène). 12 g de propylène sont alors introduits. L'agitation est alors mise en route (t=0). La température est maintenue à -10°C pendant 10 min puis est amenée doucement à 10°C. La consommation du propylène est suivie par la diminution de la pression. La phase liquide est alors prélevée et neutralisée avec NaOH aqueux. La phase organique est pesée et analysée par GC équipée d'un cryostat. Les résultats figurent dans le tableau 2.

Les complexes **7, 8** et **9** après activation avec l'activateur EADC sont très actifs pour l'oligomérisation du propylène à 10°C. La sélectivité en C6 des complexes **7** et **8** est supérieure au complexe de référence **9.** La sélectivité en 1-diméthylbutène et 2-diméthylbutène est d'environ 25% pour les complexes **7** et **8** activés.

**Tableau 2: Oligomérisation du propylène avec différents complexes activés par l'activateur EADC.^{a}**

| Entrée | Complexe | Temps (min.) | Activité^{b} | Distribution en oligomères [% pds]^{c} | | | |
|---|---|---|---|---|---|---|---|
| | | | | C6 | C9 | C12 | C15+ |
| 1^{d} | **6** | 54 | inactif | | | | |
| 2 | **7** | 30 | 4 | 96,8 | 2,9 | 0,2 | 0,1 |
| 3 | **8** | 5 | 24 | 97,1 | 2,3 | 0,2 | 0,4 |
| 4^{d} | **9** | 42 | 2,9 | 86,4 | 12,1 | 1,3 | 0,2 |

| | | | | | | | |
|---|---|---|---|---|---|---|---|
| ^{a} Conditions de réaction : nₙᵢ = 10 µmol, co-catalyseur : EADC (15 éq.), 20g C₃H₆, 10°C, solvant : chlorobenzene (50 mL). ^{b} 10⁶ g_{oligo}.mol_{Ni}^{-h}.h⁻¹. ^{c} Déterminé par GC avec le n-heptane comme étalon interne. ^{d} Exemple comparatif. | | | | | | | |

La sélectivité en dimères obtenue avec les complexes **7, 8** et **9** activés avec l'activateur EADC est reportée dans le tableau 3.

**Tableau 3: sélectivité en dimères**

| Entrée | Complexe | 4M1P | 1-DMB | 4M2P | 2M1P | 2M2P | Hex | 2-DMB |
|---|---|---|---|---|---|---|---|---|
| 2 | **7** | 1,2 | 23,7 | 35,5 | 13 | 13,4 | 11,4 | 1,8 |
| 3 | **8** | 1,1 | 17,3 | 43,3 | 12,4 | 11 | 13,6 | 1,3 |
| 4^{a} | **9** | 6,6 | 62,2 | 10,9 | 17,2 | 0,5 | 2,4 | 0,2 |

| | | | | | | | | |
|---|---|---|---|---|---|---|---|---|
| Sélectivité en dimère en % pds, déterminé par GC. 4M1 P : 4-méthylpentène-1,1-DMB : 2,3-diméthylbutène-1, 4M2P : 4-méthylpentène-2, 2M1P : 2-méthylpentène-1, 2M2P : 2-méthylepentène-2, Hex : hexènes linéaires, 2-DMB : 2,3-diméthylbutène-2. ^{a} Exemples comparatifs. | | | | | | | | |

### Tests avec MAO

Conditions opératoires : Le réacteur de 250 mL est séché sous vide à 100°C pendant 2 heures, refroidi à 10°C puis rempli de propylène (pression de 1,4 bar). 33 mL de chlorobenzène et 10 mL of n-heptane (étalon interne pesé avec précision) sont alors introduits, suivis de 4 g de propylène. Le cocatalyseur MAO (1.5 M in toluène, 300 éq, 2 mL) est alors injecté suivi du catalyseur (0.1 mmol dans 5 mL de chlorobenzène). 16 g de propylène sont alors introduits. L'agitation est alors mise en route (t=0). La température est maintenue à 10°C pendant 10 min puis est amenée doucement à 45°C. La consommation du propylène est suivie par la diminution de la pression. La phase liquide est alors prélevée et neutralisée avec H₂SO₄ aqueux. La phase organique est pesée et analysée par GC équipée d'un cryostat. Les résultats figurent dans le tableau 4.

Les complexes **7, 8** et **9** après activation avec MAO sont actifs pour l'oligomérisation du propylène à 45°C. La sélectivité en C6 des complexes **7** et **8** est supérieure au complexe de référence **9.** La sélectivité en 1-diméthylbutène et 2-diméthylbutène (DMB 1 et 2) est de 40,7% et de 47,8% pour les complexes **7** et **8** activés, respectivement.

**Tableau 4: Oligomérisation du propylène avec différents complexes activés par le MAO.^{a}**

| Entrée | Complexe | Distribution en oligomères [% pds]^{b} | | | |
|---|---|---|---|---|---|
| | | C6 | C9 | C12 | C15+ |
| 1 | **7** | 81.3 | 12,3 | 3,9 | 2,5 |
| 2 | **8** | 80.1 | 14 | 4.3 | 1,6 |
| 3^{c} | **9** | 56,5 | 23,3 | 10,8 | 9,4 |

| | | | | | |
|---|---|---|---|---|---|
| ^{a} Conditions de réaction : nₙᵢ =10 µmol, co-catalyseur : MAO (300 éq.), 10°C, 20g C₃H₆, solvant : chlorobenzene (50 mL), temps de réaction : 110 min, conversion totale. ^{b} Déterminé par GC avec le n-heptane comme étalon interne. ^{c} Exemples comparatifs. | | | | | |

La sélectivité en dimères obtenue avec les complexes **7, 8** et **9** activés avec l'activateur MAO est reportée dans le tableau 5.

**Tableau 5: Sélectivité en dimères.**

| Entrée | Complexe | 4M1P | 1-DMB | 4M2P | 2M1P | 2M2P | Hex | 2-DMB |
|---|---|---|---|---|---|---|---|---|
| 1 | **7** | 1,9 | 37,9 | 11,8 | 25,3 | 13,9 | 6,4 | 2,8 |
| 2 | **8** | 1,8 | 44,7 | 7,8 | 25,5 | 12,3 | 4,8 | 3,1 |
| 3^{a} | **9** | 0,6 | 72,8 | 6,3 | 12,9 | 4,1 | 2,4 | 0,9 |

| | | | | | | | | |
|---|---|---|---|---|---|---|---|---|
| Sélectivité en dimère en % pds, déterminé par GC. 4M1P : 4-méthylpentène-1,1-DMB : 2,3-diméthylbutène-1, 4M2P : 4-méthylpentène-2, 2M1P : 2-méthylpentène-1, 2M2P : 2-méthylepentène-2, Hex : hexènes linéaires, 2-DMB : 2,3-diméthylbutène-2. ^{a} Exemples comparatifs. | | | | | | | | |

Les exemples ci-dessus démontrent que les complexes catalytiques du procédé selon l'invention présentent une activité et une sélectivité améliorées pour l'oligomérisation des oléfines comportant de préférence 2 à 10 atomes de carbone, plus particulièrement pour la dimérisation des oléfines comportant de 2 à 10 atomes de carbone.

## Revendications

1. Complexe dissymétrique de nickel de formule (I) : 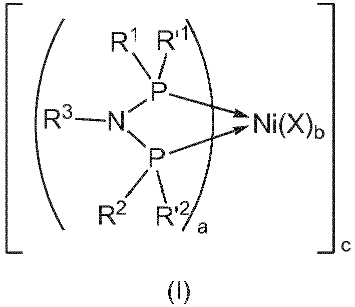 dans lequel
- les groupements R¹ et R'¹, identiques ou différents, liés ou non entre eux, sont choisis parmi les groupements non aromatiques et ne contenant pas de silicium,
- les groupements R² et R'², identiques ou différents, liés ou non entre eux, sont choisis parmi les groupements aromatiques,
- R³ est choisi parmi l'hydrogène, les halogènes, les groupements hydrocarbonés aliphatiques, cycliques ou non, contenant ou non des hétéroéléments, les groupements aromatiques contenant ou non des hétéroéléments, substitués ou non,
- X est un anion ou un donneur d'électrons, les groupements X peuvent être liés entre eux ou non, X est choisi parmi l'hydrogène, les halogènes, les groupements hydrocarbonés aliphatiques, cycliques ou non, contenant ou non des hétéroéléments substitués ou non, les groupements aromatiques contenant ou non des hétéroéléments, substitués ou non, les oléfines contenant ou non des hétéroéléments, substituées ou non, les borates, les phosphates, les sulfates, les ligands phosphorés contenant ou non des hétéroéléments, substitués ou non, les groupements -OR⁴ ou -N(R⁵)(R⁶), où R⁴, R⁵ et R⁶ sont choisis parmi les groupements hydrocarbonés aliphatiques, cycliques, contenant ou non des hétéroéléments, les groupements aromatiques contenant ou non des hétéroéléments, substitués ou non,
- a est un nombre entier compris entre 1 et 4, b est un nombre entier compris entre 0 et 6, c est un nombre entier compris entre 1 et 4.

2. Complexe selon la revendication 1 dans lequel les groupements R¹ et R'¹ sont choisis parmi les groupements méthyle, éthyle, isopropyle, *n*-butyle, *iso*-butyle, *tert-*butyle, pentyle, cyclohexyle, substitués ou non.

3. Complexe selon l'une des revendications 1 à 2 dans lequel les groupements R² et R'² sont choisis parmi les groupements phényle, o-tolyl, m-tolyl, *p*-tolyl, mésityle, 3,5-diméthylphényle, 4-méthoxyphényle, 2-méthoxyphényle, 2-isopropoxyphényle, 4-méthoxy-3,5-diméthylphényle, 3,5-ditert-butyl-4-méthoxyphényle, 3,5-bis(trifluorométhyl)phenyle, benzyle, naphthyle, pyridyle, substitués ou non, contenant des hétéro-éléments ou non.

4. Procédé de préparation des complexes selon l'une des revendications 1 à 3 comprenant la mise en contact d'un précurseur de nickel A et au moins un ligand diphosphinamine B1 de formule (R¹)(R'¹)P-N(R³)-P(R²)(R'²) ou au moins un ligand iminobisphosphine B2 de formule (R³)N=P(R¹)(R'¹)-P(R²)(R'²) en présence ou pas d'un solvant, R¹, R'¹, R², R'² et R³ répondant aux spécifications selon l'une des revendications 1 à 4.

5. Procédé de préparation selon la revendication 4 mis en oeuvre à une température comprise entre -80°C et +110°C, pendant une durée comprise entre 1 minute et 24 heures.

6. Procédé de préparation selon la revendication 4 ou 5 dans lequel le précurseur de nickel A est choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), le nickel bis(éthylène), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydraté ou non, pris seul ou en mélange. Lesdits précurseurs de nickel peuvent éventuellement être complexés à des bases de Lewis.

7. Procédé d'oligomérisation d'une charge d'oléfines comprenant la mise en contact de ladite charge avec le complexe selon l'une des revendications 1 à 3 ou préparé selon les revendications 4 à 6, en présence ou pas de solvant.

8. Procédé selon la revendication 7 dans lequel le complexe est utilisé en mélange avec un composé C choisi dans le groupe formé par les composés tris(hydrocarbyl)aluminium, les composés chlorés ou bromés d'hydrocarbylaluminium, les aluminoxanes, les composés organo-borés, les composés organiques susceptibles de donner ou capter un proton, pris seuls ou en mélange.

9. Procédé selon l'une des revendications 7 à 8 dans lequel les oléfines sont choisies parmi l'éthylène, le propylène, les n-butènes et les n-pentènes, seules ou en mélange, pures ou diluées.

10. Procédé selon l'une des revendications 7 à 9 dans lequel le nickel est présent dans une concentration comprise entre 1.10⁻⁸ et 1 mol/L.

11. Procédé selon l'une des revendications 7 à 10 dans lequel on opère à une pression totale comprise entre la pression atmosphérique et 20 MPa et à une température comprise entre -40 et +250°C.

12. Procédé selon l'une des revendications 7 à 11 dans lequel la réaction est une réaction de dimérisation.

13. Procédé selon la revendication 12 dans lequel la réaction est une réaction de dimérisation de l'éthylène ou du propylène.

## Patentansprüche

1. Asymmetrischer Nickel-Komplex der Formel (I): 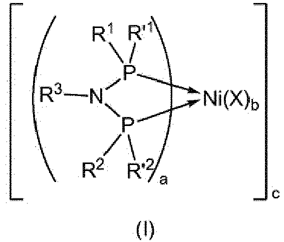 wobei
- die Gruppen R¹ und R'¹, identisch oder verschieden, miteinander verbunden oder nicht, aus nicht-aromatischen Gruppen ausgewählt sind und kein Silicium enthalten,
- die Gruppen R² und R'², identisch oder verschieden, miteinander verbunden oder nicht, aus aromatischen Gruppen ausgewählt sind,
- R³ausgewählt ist aus Wasserstoff, Halogenen, aliphatischen Kohlenwasserstoff-Gruppen, cyclisch oder nicht, enthaltend Heteroelemente oder nicht, aromatischen Gruppen, enthaltend Heteroelemente oder nicht, substituiert oder nicht,
- X ein Anion oder ein Elektronendonator ist, die Gruppen X miteinander verbunden sein können oder nicht, X ausgewählt ist aus Wasserstoff, Halogenen, aliphatischen Kohlenwasserstoff-Gruppen, cyclisch oder nicht, enthaltend Heteroelemente oder nicht, substituiert oder nicht, aromatischen Gruppen, enthaltend Heteroelemente oder nicht, substituiert oder nicht, Olefinen, enthaltend Heteroelemente oder nicht, substituiert oder nicht, Boraten, Phosphaten, Sulfaten, Phosphor-Liganden, enthaltend Heteroelemente oder nicht,substituiert oder nicht, Gruppen -OR⁴ oder -N(R⁵)(R⁶), wobei R⁴, R⁵ und R⁶ ausgewählt sind aus aliphatischen, cyclischen Kohlenwasserstoff-Gruppen, enthaltend Heteroelemente oder nicht, aromatischen Gruppen, enthaltend Heteroelemente oder nicht, substituiert oder nicht,
- a eine ganze Zahl zwischen 1 und 4 ist, b eine ganze Zahl zwischen 0 und 6 ist, c eine ganze Zahl zwischen 1 und 4 ist.

2. Komplex nach Anspruch 1, wobei die Gruppen R¹ und R'¹ausgewählt sind aus Methyl-, Ethyl-, Isopropyl-, n-Butyl-, Isobutyl-, tert-Butyl-, Pentyl-, Cyclohexyl-Gruppen, substituiert oder nicht.

3. Komplex nach einem der Ansprüche 1 bis 2, wobei die Gruppen R² und R'²ausgewählt sind aus Phenyl-, o-Tolyl-, m-Tolyl-, p-Tolyl-, Mesityl-, 3,5-Dimethylphenyl-, 4-Methoxyphenyl-, 2-Methoxyphenyl-, 2-Isopropoxyphenyl-, 4-Methoxy-3,5-dimethylphenyl-, 3,5-Di-tert-butyl-4-methoxyphenyl-, 3,5-Bis-(trifluormethyl)-phenyl-, Benzyl-, Naphthyl-, Pyridyl-Gruppen, substituiert oder nicht, enthaltend Heteroelemente oder nicht.

4. Verfahren zur Herstellung von Komplexen nach einem der Ansprüche 1 bis 3, umfassend das Inberührungbringen eines Nickel-Vorläufers A und mindestens eines Diphosphinamin-Liganden B1 der Formel (R¹)(R'¹)P-N(R³)-P(R²)(R'²) oder mindestens eines Iminobisphosphin-Liganden B2 der Formel (R³)N=P(R¹)(R'¹)-P(R²)(R'²) in Anwesenheit eines Lösungsmittels oder nicht, wobei R¹, R'¹, R², R'² und R³ die Bedeutungen nach einem der Ansprüche 1 bis 4 haben.

5. Herstellungsverfahren nach Anspruch 4, welches bei einer Temperatur zwischen -80 °C und +110 °C, während einer Dauer zwischen 1 Minute und 24 Stunden, durchgeführt wird.

6. Herstellungsverfahren nach Anspruch 4 oder 5, wobei der Nickel-Vorläufer A ausgewählt wird aus Nickel(II)-chlorid, Nickel(II)-(dimethoxyethan)-chlorid, Nickel(II)-bromid, Nickel(II)-(dimethoxyethan)-bromid, Nickel(II)-fluorid, Nickel(II)-iodid, Nickel(II)-sulfat, Nickel(II)-carbonat, Nickel(II)-dimethylglyoxim, Nickel(II)-hydroxid, Nickel(II)-hydroxyacetat, Nickel(II)-oxalat, Nickel(II)-carboxylaten, wie beispielsweise 2-Ethylhexanoat, Nickel(II)-phenaten, Nickel(II)-acetat, Nickel(II)-trifluoracetat, Nickel(II)-triflat, Nickel(II)-acetylacetonat, Nickel(II)-hexafluoracetylacetonat, Nickel(0)-bis-(cycloocta-1,5-dien), Nickel(0)-bis-(cycloocta-1,3-dien), Nickel(0)-bis-(cyclooctatetraen), Nickel(0)-bis-(cycloocta-1,3,7-trien), Bis-(o-tolylphosphito)-nickel(0)-(ethylen), Nickel(0)-tetrakis-(triphenylphosphit), Nickel(0)-tetrakis-(triphenylphosphin), Nickel-bis-(ethylen), Π-Allylnickel(II)-chlorid, Π-Allylnickel(II)-bromid,Methallylnickel(II)-chloriddimer, η³-Allylnickel(II)-hexafluorphosphat,η³-Methallylnickel(II)-hexafluorphosphat und Nickel(II)-1,5-cyclooctadien, in ihrer hydratisierten Form oder nicht, allein oder in Mischung. Die Nickel-Vorläufer können gegebenenfalls Komplexe mit Lewis-Basen sein.

7. Verfahren zur Oligomerisation einer Olefin-Charge, umfassend das Inberührungbringen der Charge mit dem Komplex nach einem der Ansprüche 1 bis 3, oder hergestellt nach den Ansprüchen 4 bis 6, in Anwesenheit eines Lösungsmittels oder nicht.

8. Verfahren nach Anspruch 7, wobei der Komplex in Mischung mit einer Verbindung C verwendet wird, die ausgewählt wird aus der Gruppe bestehend aus Tris-(hydrocarbyl)-aluminium-Verbindungen, Chlor- oder Brom-Verbindungen von Hydrocarbylaluminium, Aluminoxanen, Organobor-Verbindungen, organischen Verbindungen, die ein Proton abgeben oder einfangen können, allein oder in Mischung.

9. Verfahren nach einem der Ansprüche 7 bis 8, wobei die Olefine ausgewählt werden aus Ethylen, Propylen, n-Butenen und n-Pentenen, allein oder in Mischung, rein oder verdünnt.

10. Verfahren nach einem der Ansprüche 7 bis 9, wobei das Nickel in einer Konzentration zwischen 1.10⁻⁸ und 1 mol/l vorhanden ist.

11. Verfahren nach einem der Ansprüche 7 bis 10, wobei bei einem Gesamtdruck zwischen Atmosphärendruck und 20 MPa und bei einer Temperatur zwischen -40 und +250 °C gearbeitet wird.

12. Verfahren nach einem der Ansprüche 7 bis 11, wobei die Reaktion eine Dimerisierungsreaktion ist.

13. Verfahren nach Anspruch 12, wobei die Reaktion eine Dimerisierungsreaktion von Ethylen oder Propylen ist.

## Claims

1. Dissymmetric nickel complex of formula (I): 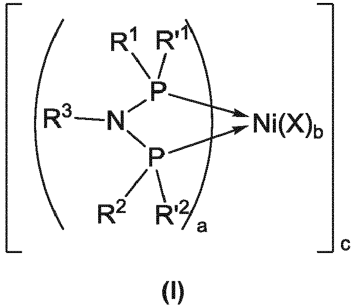 in which
- the groups R¹ and R'¹, which may be identical or different, and may or may not be linked, are selected from the non-aromatic groups not containing silicon,
- the groups R² and R'², which may be identical or different, and may or may not be linked, are selected from the aromatic groups,
- R³ is selected from hydrogen, the halogens, the aliphatic hydrocarbon groups, cyclical or not, and which may or may not contain heteroelements, and the aromatic groups which may or may not contain heteroelements, which may or may not be substituted,
- X is an anion or an electron donor, the groups X may or may not be linked, X is selected from hydrogen, the halogens, the aliphatic hydrocarbon groups, cyclical or not, and which may or may not contain heteroelements, which may or may not be substituted, and the aromatic groups which may or may not contain heteroelements, which may or may not be substituted, the olefins, which may or may contain heteroelements, which may or may not be substituted, the borates, the phosphates, the sulphates, the phosphorous ligands which may or may not contain heteroelements, which may or may not be substituted, the -OR⁴ or -N(R⁵)(R⁶) groups, where R⁴, R⁵ and R⁶ are selected from the aliphatic hydrocarbons groups, cyclical, which may or may not contain heteroelements, the aromatic groups which may or may not contain heteroelements, which may or may not be substituted,
- a is a whole number between 1 and 4, b is a whole number between 0 and 6, and c is a whole number between 1 and 4.

2. Complex according to claim 1 in which the groups R¹ and R'¹ are selected from methyl, ethyl, isopropyl, n-butyl, iso-butyl, tert-butyl, pentyl, cyclohexyl groups, which may or may not be substituted or unsubstituted.

3. Complex according to one of claims 1 to 2 in which the groups R² and R'² are selected from phenyl, o-tolyl, m-tolyl, p-tolyl, mesityl, 3,5-dimethylphenyl, 4-methoxyphenyl, 2-methoxyphenyl, 2-isopropoxyphenyl, 4-methoxy-3,5-dimethylphenyl, 3,5-ditert-butyl-4-methoxyphenyl, 3,5-bis(trifluoromethyl)phenyl, benzyl, naphthyl and pyridyl, which may or may not be substituted and which may or may not contain heteroelements.

4. Method of preparation of the complexes according to one of claims 1 to 3 comprising bringing into contact a nickel precursor A and at least one diphosphinamine ligand B1 of formula (R¹)(R'¹)P-N(R³)-P(R²)(R'²) or at least one iminobisphosphine ligand B2 of formula (R³)N=P(R¹)(R'¹)-P(R²)(R'²) in the presence or not of a solvent, R¹, R'¹, R², R'² and R³ meeting the specifications according to one of claims 1 to 4.

5. Method of preparation according to claim 4 implemented at a temperature of between -80°C and +110°C, for a period of between 1 minute and 24 hours.

6. Method of preparation according to claim 4 or 5 in which the nickel precursor A is selected from nickel (II) chloride, nickel(II)(dimethoxyethane) chloride, nickel(II) bromide, nickel(II)(dimethoxyethane) bromide, nickel(II) fluoride, nickel(II) iodide, nickel(II) sulphate, nickel(II) carbonate, nickel(II) dimethylglyoxime, nickel(II) hydroxide, nickel(II) hydroxyacetate, nickel(II) oxalate, nickel(II) carboxylates such as 2-ethylhexanoate, for example, nickel(II) phenates, nickel(II) acetate, nickel(II) trifluoroacetate, nickel(II) triflate, nickel(II) acetylacetonate, nickel(II) hexafluoroacetylacetonate, nickel(0) bis(cycloocta-1,5-diene), nickel(0) bis(cycloocta-1,3-diene), nickel(0) bis(cyclooctatetraene), nickel(0) bis(cycloocta-1,3,7-triene), bis(o-tolylphosphito) nickel(0)(ethylene), nickel(0) tetrakis(triphenylphosphite), nickel(0) tetrakis(triphenylphosphine), nickel (0) bis(ethylene), π-allylnickel(II) chloride, π-allylnickel(II) bromide, methallylnickel(II) chloride dimer, η³-allylnickel(II) hexafluorophosphate, η³-methallylnickel(II) hexafluorophosphate, and nickel(II) (1,5-cyclooctadiene) in their hydrated or non-hydrated form, used alone or as a mixture. Said nickel precursors may optionally be complexed with Lewis bases.

7. Method of oligomerisation of a feed of olefins comprising bringing said feed into contact with the complex according to one of claims 1 to 3 or prepared according to claims 4 to 6, whether or not a solvent is present.

8. Method according to claim 7 in which the complex is used in a mixture with a compound C selected from the group formed by tris(hydrocarbyl)aluminium compounds, chlorine-containing or bromine-containing hydrocarbylaluminium compounds, aluminoxanes, organo-boron compounds, and organic compounds which are susceptible of donating or accepting a proton, used alone or as a mixture.

9. Method according to one of claims 7 to 8 in which the olefins are selected from ethylene, propylene, the n-butenes and the n-pentenes, used alone or in a mixture, pure or diluted.

10. Method according to one of claims 7 to 9 in which the nickel is present in a concentration of between 1x10-8 and 1 mol/l.

11. Method according to one of claims 7 to 10 in which a total pressure is operated at in the range between atmospheric pressure and 20 MPa, and at a temperature in the range -40° C. to +250° C.

12. Method according to one of claims 7 to 11 in which the reaction is a dimerisation reaction.

13. Method according to claim 12 in which the reaction is an ethylene or propylene dimerisation reaction.
